(19) **Europäisches Patentamt**
**European Patent Office**
**Office européen des brevets**

(11) **EP 3 142 543 B1**

(12) **EUROPEAN PATENT SPECIFICATION**

(45) Date of publication and mention
of the grant of the patent:
**11.12.2019 Bulletin 2019/50**

(21) Application number: **15724378.3**

(22) Date of filing: **12.05.2015**

(51) Int Cl.:
*A61B 5/00* *(2006.01)*  *G01N 29/24* *(2006.01)*
*G01N 21/17* *(2006.01)*

(86) International application number:
**PCT/JP2015/002411**

(87) International publication number:
**WO 2015/174082 (19.11.2015 Gazette 2015/46)**

(54) **PHOTOACOUSTIC APPARATUS**

PHOTOAKUSTISCHE VORRICHTUNG

APPAREIL PHOTO-ACOUSTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2014 US 201461993132 P**

(43) Date of publication of application:
**22.03.2017 Bulletin 2017/12**

(73) Proprietor: **Canon Kabushiki Kaisha
Tokyo 146-8501 (JP)**

(72) Inventors:
• **NANAUMI, Ryuichi
Tokyo 146-8501 (JP)**

• **MIYASATO, Takuro
Tokyo 146-8501 (JP)**
• **KRUGER, Robert A.
Tokyo 146-8501 (JP)**

(74) Representative: **Williamson, Brian et al
Canon Europe Limited
European Patent Department
3 The Square
Stockley Park
Uxbridge, Middlesex UB11 1ET (GB)**

(56) References cited:
**WO-A1-2013/063539    US-A1- 2014 036 636**

**Description**

**Technical Field**

The present invention relates to a photoacoustic apparatus.

**Background Art**

Photoacoustic apparatuses such as photoacoustic imaging apparatuses and ultrasonic echo imaging apparatuses have been hitherto proposed as devices based on technology for acoustic imaging of target objects being examined, such as living bodies, in which acoustic waves are received from inside the target objects to obtain information on the inside of the target objects.

For example, photoacoustic imaging apparatuses are profitable for use in especially the diagnosis of skin cancer or breast cancer. Expectations are increasingly growing for photoacoustic imaging apparatuses to be used as medical devices that replace existing apparatuses for use in such diagnosis, such as ultrasonic echo diagnostic apparatuses, X-ray apparatuses, and magnetic resonance imaging (MRI) apparatuses.

Irradiation of biological tissue with measurement light such as visible light or near-infrared light causes instantaneous expansion of a light-absorbing substance inside the biological tissue, for example, a substance in blood, such as hemoglobin, by absorbing the energy of the measurement light, resulting in the generation of an acoustic wave. This phenomenon is called a photoacoustic effect, and the generated acoustic wave is also referred to as a photoacoustic wave.

A photoacoustic imaging apparatus measures such a photoacoustic wave to visualize information on the biological tissue. Technology for tomographic imaging based on such a photoacoustic effect is also referred to as photoacoustic imaging (PAI).

Photoacoustic imaging enables the imaging of information related to the absorption coefficient of the inside of the target object. The absorption coefficient is a ratio of the optical energy absorbed by biological tissue to the optical energy incident on the biological tissue. Examples of the information related to the absorption coefficient include initial sound pressure, which is the sound pressure at the moment of generation of a photoacoustic wave. The initial sound pressure is proportional to the product of the optical energy and the absorption coefficient. Thus, an initial sound pressure value is subjected to appropriate processing to determine an absorption coefficient.

In addition, the absorption coefficient depends on the concentration of the components of the biological tissue. Accordingly, the concentration of the components can be obtained based on the absorption coefficient. In particular, using the light of a wavelength more likely to be absorbed by the hemoglobin in the blood, the ratio of oxygenated hemoglobin to deoxygenated hemoglobin concentration and, in addition, the oxygen saturation of the biological tissue can be obtained. It is anticipated that analysis of an oxygen saturation distribution will be applied to medical diagnosis such as in the case of identifying tumor tissue in a living body or tissue surrounding the tumor.

PTL 1 discloses a technique for forming a photoacoustic image from reception signals obtained at a plurality of positions by scanning both a light source that irradiates a target object with light and electromechanical conversion elements that receive photoacoustic waves.

[Citation List]

[Patent Literature]

PTL 1:
Japanese Patent Laid-Open No. 2010-22812
US2014036636 describes an object information acquiring apparatus including an irradiation unit irradiating an object with light; a detection unit including a plurality of elements each obtaining an acoustic wave generated from the object and converting the acoustic wave into a detection signal; a detection unit moving mechanism moving the detection unit to the object relatively; an adding unit selecting, from detection signals converted from acoustic waves obtained by the plurality of elements at respective positions as the detection unit is moved, detection signals converted from acoustic waves obtained from an identical position on the object, and adding together these detection signals to output a resultant summed signal; an initial sound pressure computing unit computing an initial sound pressure of a region of interest from the summed signal; a light quantity computing unit computing a quantity of light of the region of interest.

[Summary of Invention]

An aspect of the present invention provides a photoacoustic apparatus according to claim 1. Another aspect of the present invention provides a signal processing method according to claim 12. A further aspect of the present invention provides a computer program according to claim 13.

[Brief Description of Drawings]

[Fig. 1] Fig. 1 is a schematic diagram of a photoacoustic apparatus according to a first embodiment.
[Fig. 2] Fig. 2 is a diagram illustrating the configuration of a signal processing unit according to the first embodiment.
[Fig. 3] Fig. 3 is a flow diagram of a target-object information acquisition method according to the first embodiment.
[fig.4A]Fig. 4A is a diagram illustrating the scanning of a probe and an optical system according to the first embodiment.
[fig.4B]Fig. 4B is a diagram illustrating the scanning of the probe and the optical system according to the first embodiment.
[fig.5A]Fig. 5A is a diagram illustrating the light fluence in a target object at scanning positions according to the first embodiment.
[fig.5B]Fig. 5B is a diagram illustrating the light fluence in the target object at the scanning positions according to the first embodiment.
[fig.6]Fig. 6 is a schematic diagram of a photoacoustic apparatus according to a second embodiment.
[fig.7A]Fig. 7A is a diagram illustrating the details of a probe according to the second embodiment.
[fig.7B]Fig. 7B is a diagram illustrating the details of the probe according to the second embodiment.
[fig.7C]Fig. 7C is a diagram illustrating the details of the probe according to the second embodiment.
[fig.7D]Fig. 7D is a diagram illustrating the details of the probe according to the second embodiment.
[fig.8A]Fig. 8A is a diagram illustrating the scanning of the probe over the x-y plane according to the second embodiment, viewed in the z-direction.
[fig.8B]Fig. 8B is a diagram illustrating the scanning of the probe over the x-y plane according to the second embodiment, viewed in the z-direction.
[fig.8C]Fig. 8C is a diagram illustrating the scanning of the probe over the x-y plane according to the second embodiment, viewed in the z-direction.
[fig.9]Fig. 9 is a diagram of the scanning of the probe over the x-y plane according to the second embodiment, viewed in the y-direction.
[fig. 10] Fig. 10 is a diagram of a computational model to illustrate the effect of Example.
[fig. 11A] Fig. 11A is a diagram illustrating the reduction in artifacts in Example.
[fig. 11B]Fig. 11B is a diagram illustrating the reduction in artifacts in Example.

**Description of Embodiments**

Some exemplary embodiments of the present invention will be described hereinafter with reference to the drawings. Note that the dimensions, materials, shapes, relative positions, and so forth of components provided in the following description may vary, as appropriate, depending on various conditions and the configuration of an apparatus to which an embodiment of the invention is applied and are not intended to limit the scope of the invention to the following embodiments.

The present invention relates to a technique for detecting a propagating acoustic wave from a target object being examined (hereinafter referred to as the "target object") and generating and obtaining target-object information on the inside of the target object. Thus, aspects of the present invention may provide a photoacoustic apparatus and a control method therefor, a target-object information acquisition method, and a signal processing method. Further aspects of the present invention may provide a program for causing an information processing apparatus including hardware resources such as a central processing unit (CPU) to perform the methods described above, and a storage medium storing the program.

Photoacoustic apparatuses according to aspects of the present invention include an apparatus that utilizes photoacoustic tomography technology for irradiating a target object with light (electromagnetic radiation) and receiving (or detecting) acoustic waves generated and propagated in the target object or at specific positions on a surface of the target object in accordance with the photoacoustic effect. Such a photoacoustic apparatus obtains the target-object information on the inside of the target object in the form of image data or the like based on photoacoustic measurement, and is thus referred to also as a photoacoustic imaging apparatus.

The target-object information obtained in the photoacoustic apparatus indicates a source distribution of acoustic waves produced by irradiation of the target object with light, or an initial sound pressure distribution in the target object. Alternatively, the target-object information indicates an optical energy absorption density distribution or an absorption coefficient distribution, which may be derived from an initial sound pressure distribution, or a concentration distribution of the substances constituting tissue. Specific examples of the target-object information include a blood component distribution such as distributions of oxygenated hemoglobin and deoxygenated hemoglobin concentrations or an oxygen saturation distribution determined using the distributions of oxygenated hemoglobin and deoxygenated hemoglobin concentrations, and a distribution of fat, collagen, and water content.

As used herein, the term "acoustic wave" typically refers to an ultrasonic wave, and is used to include an elastic wave called a sound wave or an acoustic wave. An acoustic wave produced by using the photoacoustic effect is referred to as a photoacoustic wave or an optical ultrasonic wave. An electrical signal converted from an acoustic wave by using a probe is also referred to as an acoustic signal.

In this specification, a plurality of time-series reception signals respectively output from a plurality of transducers when a target object and a position at which the target object is irradiated with light (hereinafter referred to as an "irradiation position") are in a certain relative positional relationship are collectively referred to as a time-series reception signal group.

## First Embodiment

An embodiment of the present invention will be described in detail hereinafter with reference to the drawings. The same or similar components are basically given the same numerals and are not described herein.

## Configuration of Photoacoustic Apparatus according to First Embodiment

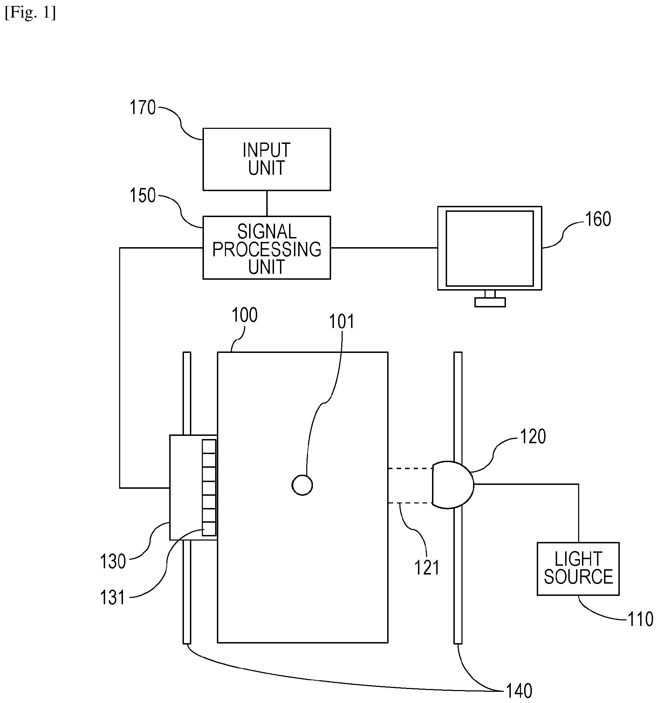

Fig. 1 is a schematic diagram of a photoacoustic apparatus. A description will now be given of each of the components of the photoacoustic apparatus. The photoacoustic apparatus includes a light source 110, an optical system 120, a probe 130 including a plurality of transducers 131, a scanning unit 140, a signal processing unit 150, a display unit 160, and an input unit 170. A target object 100 is a target of measurement. The scanning unit 140 scans the probe 130 and the optical system 120 over the target object 100.

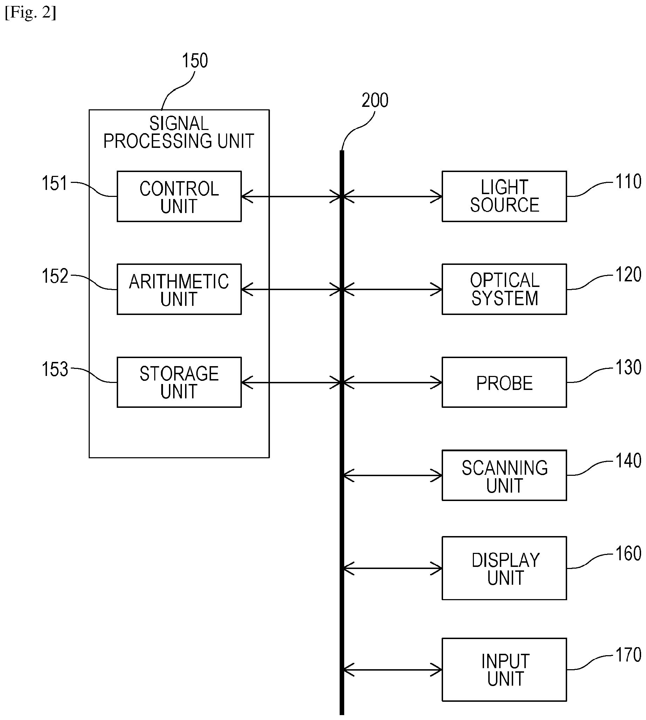

Fig. 2 is a schematic diagram illustrating the details of the signal processing unit 150 and the configuration of components around the signal processing unit 150. The signal processing unit 150 includes a control unit 151, an arithmetic unit 152, and a storage unit 153.

The control unit 151 controls the operation of the components of the photoacoustic apparatus via a bus 200. Further, the control unit 151 loads a program that describes a target-object information acquisition method, described below, which is stored in the storage unit 153, into the arithmetic unit 152 to cause the photoacoustic apparatus to implement the target-object information acquisition method.

The storage unit 153 stores the program that describes the target-object information acquisition method. Also, the storage unit 153 temporarily stores input and output data from the respective units to perform the imaging operation of the overall apparatus so as to enable exchange of data between the respective units. Note that each unit may include a data storage unit to perform individual processing separately from the storage unit 153.

First, light generated from the light source 110 passes through the optical system 120, and the target object 100 is irradiated with the light as pulsed light 121. Due to the photoacoustic effect, photoacoustic waves are produced in the target object 100. Thereafter, the probe 130 receives the propagating acoustic waves, and obtains a time-series reception signal group. This location is referred to as a first scanning position.

Then, the scanning unit 140 scans the optical system 120 and the probe 130 to perform the series of measurement operations described above at a second scanning position, and stores a reception signal in the storage unit 153. When measurement is performed at all the scanning positions and the storage of reception signal groups in the storage unit 153 is completed, the signal processing unit 150 generates target-object information based on a reception signal group for each scanning position, which is stored in the storage unit 153, and causes the target-object information to be displayed on the display unit 160.

Imaging of a region of interest including a light absorber 101 by using the reception signals at the individual scanning positions results in artifacts occurring in portions within the region of interest where the light absorber 101 is not present. The signal processing unit 150 suppresses the occurrence of artifacts by, when imaging minimum units constituting the region of interest, weighting a weighting factor to reception signals for each scanning position, based on a light fluence in a minimum unit in which the respective reception signals are obtained. Furthermore, target-object information on the region of interest, that is, the initial sound pressure or absorption coefficient, is acquired through processing that takes into account a weight factor based on the light fluence to obtain target-object information with high quantitativity.

The components of the photoacoustic apparatus according to this embodiment will be described in detail.

## Target Object 100 and Light Absorber 101

The target object 100 and the light absorber 101 will now be described although they are not part of the photoacoustic apparatus according to this embodiment of the present invention. The photoacoustic apparatus according to this embodiment of the present invention is designed for use mainly in diagnosis of malignant tumor or diseases such as a vascular disease in humans or animals, follow-up during chemotherapy, and the like. Thus, the target object is assumed to be a living body, more specifically, a body part to be diagnosed, such as within the breast, neck, or abdomen of a human or an animal.

It is also assumed that the light absorber inside the target object is an object of a relatively high optical absorption coefficient inside the target object. For example, in a case where a human body is a target of measurement, oxyhemoglobin or deoxyhemoglobin, a blood vessel including a large amount of oxyhemoglobin or deoxyhemoglobin, or a malignant tumor including a large number of new blood vessels is a light absorber which is a target of measurement. In addition, plaque depositions in the carotid artery wall and the like are also targets of measurement.

**Light Source 110**

The light source 110 is preferably a pulsed light source capable of generating pulsed light on the order of several nanoseconds to several microseconds. Specifically, in terms of efficient generation of photoacoustic waves, it is preferable that the light source 110 be capable of generating light having a pulse width of approximately 10 nanoseconds. The light generated by the light source 110 desirably has a wavelength that allows light to propagate through the inside of the target object. Specifically, in a case where the target object is a living body, the wavelength preferably ranges from 500 nm to 1200 nm, both inclusive. If an optical characteristic value distribution of biological tissue comparatively near a surface of the living body is to be determined, a wider wavelength region (of, for example, 400 nm to 1600 nm) than the wavelength region described above may be used.

The light source 110 may be a laser and a light-emitting diode. The laser may be of any of various types, including, for example, a solid-state laser, a gas laser, a dye laser, and a semiconductor laser. Examples of a laser that may be used in this embodiment include an alexandrite laser, an yttrium-aluminium-garnet laser, and a Titan-Sapphire laser.

**Optical System 120**

The light emitted from the light source 110 is formed into a desired light distribution shape by the optical system 120, and is guided into the target object 100. The optical system 120 typically includes optical components such as a lens and a mirror. The light may be propagated using an optical waveguide such as an optical fiber. The optical components may include, for example, a mirror that reflects light, a lens that condenses or magnifies light to change the shape of the light, a prism that disperses, refracts, and reflects light, an optical fiber that allows light to propagate, and a diffuser that diffuses light. Any optical component capable of irradiating a target object with the light emitted from the light source 110 in a desired shape may be used.

The intensity of the light with which the target object 100 is to be irradiated from the optical system 120 may be set in advance and stored in the storage unit 153. The control unit 151 drives the light source 110 to irradiate the target object 100 with illumination light at the set intensity. Alternatively, the light source 110 or the optical system 120 may be provided with a light intensity sensor. In this case, part of the light to be actually emitted may be measured to determine the intensity of the illumination light, and the intensity of the illumination light may be stored in the storage unit 153.

If it is possible to irradiate the target object 100 with, as desired light, the light emitted from the light source 110, the optical system 120 may not necessarily be used.

**Probe 130**

The probe 130 includes transducers 131, which are elements capable of detecting acoustic waves, and a housing that houses the transducers 131.

The transducers 131 receive photoacoustic waves, and convert the photoacoustic waves into electrical signals, which are analog signals. The transducers 131 may be any devices capable of receiving photoacoustic waves, such as devices based on a piezoelectric phenomenon, optical resonance, and changes in capacitance.

Frequency components of a photoacoustic wave typically range from 100 kHz to 100 MHz. It is thus preferable that devices capable of detecting such frequencies be used as the transducers 131.

The probe 130 preferably includes a plurality of transducers 131 arranged in an array. Accordingly, photoacoustic waves generated by single irradiation of the target object 100 with light can be acquired at a plurality of positions, resulting in an increase in the amount of information to be used for imaging and in an improvement in image quality.

**Scanning Unit 140**

The scanning unit 140 moves the relative positions of the probe 130 and the optical system 120 to the target object 100. The scanning unit 140 may include a drive unit for scanning, and a separate control unit for scanning control. The scanning unit 140 may be configured to output scanning position information on the probe 130 and the optical system 120 during scanning, which is used for the calculation of the light fluence distribution or the calculation of the initial sound pressure, described below. The scanning unit 140 corresponds to a movement unit of the present invention.

Scanning enables a large area in the target object 100 to be imaged as a region of interest. The transducers 131 generally have the highest reception sensitivity in the front direction thereof. The pulsed light 121 radiated from the optical system 120 is spread by scattering inside the target object 100, where the light fluence is high in the front direction in which the radiation is performed. Since the amplitude of a photoacoustic wave is proportional to the light fluence, a photoacoustic wave generated in the front direction in which the light radiation is performed is received in the front direction, in which the transducers 131 exhibit high reception sensitivity, resulting in an increased signal-to-noise (SN) ratio. It is thus desirable that the scanning unit 140 synchronously scan the probe 130 and the optical system 120 while maintaining the positional relationship between the probe 130 and the optical system 120 so that the pulsed light 121 can be radiated in the direction in which the transducers 131 exhibit high reception sensitivity.

A combination of the light source 110, the optical system 120, and the scanning unit 140 according to this embodiment corresponds to an irradiation unit of the present invention. The irradiation unit may have any configuration other than that described above so long as the irradiation unit is configured to irradiate the target object 100 with light at different positions at a plurality of timings. For example, the irradiation unit may include a plurality of light sources, and may be configured to emit light to different positions from the respective light sources.

**Signal Processing Unit 150**

As illustrated in Fig. 2, the signal processing unit 150 includes the control unit 151, the arithmetic unit 152, and the storage unit 153. The signal processing unit 150 corresponds to a processing unit of the present invention.

The control unit 151 typically includes an element such as a CPU.

The arithmetic unit 152 typically includes an element such as a CPU, a graphics processing unit (GPU), or an analog-to-digital (A/D) converter, or a circuit such as a field-programmable gate array (FPGA) or an application-specific integrated circuit (ASIC). The arithmetic unit 152 may also include a signal amplifier. The electrical signals converted from acoustic waves by the transducers 131 are analog signals, and are thus generally converted into digital signals and then amplified. The arithmetic unit 152 may not necessarily include a single element or a single circuit, and may include a plurality of elements or circuits. In addition, processing operations performed in a target-object information acquisition method may be executed by any element or circuit. A device that executes the processing operations is generally referred to as an arithmetic unit according to this embodiment.

The storage unit 153 typically includes a storage medium, examples of which include a read-only memory (ROM), a random access memory (RAM), and a hard disk. The storage unit 153 may not necessarily include a single storage medium, and may include a plurality of storage media.

Preferably, the signal processing unit 150 is configured to provide concurrent pipeline processing of a plurality of signals. This may reduce the time taken to acquire target-object information.

The individual processing operations performed in the target-object information acquisition method may be stored in the storage unit 153 as a program to be executed by the arithmetic unit 152. Note that the storage unit 153, which stores the program, is a non-transitory recording medium.

A signal processing unit and a plurality of transducers may be located in a common housing. A portion of signal processing may be performed in the signal processing unit located in the housing, and the remaining portion of the signal processing may be performed by a signal processing unit located outside the housing. In this case, the signal processing units located inside and outside the housing may be collectively referred to as a signal processing unit according to this embodiment.

**Display Unit 160**

The display unit 160 is a device configured to display target-object information output from the signal processing unit 150. The display unit 160 is typically a liquid crystal display or the like, or may be any other type of display such as a plasma display or an organic electroluminescent (EL) display or a field emission display (FED).

The display unit 160 may be provided separately from the photoacoustic apparatus according to this embodiment.

**Input Unit 170**

The input unit 170 is a member configured to allow desired information to be specified for inputting the desired information to the signal processing unit 150 by a user. Examples of the input unit 170 include a keyboard, a mouse, a touch panel, a dial, and a button. When a touch panel is used as the input unit 170, the touch panel may be one in which the display unit 160 also functions as the input unit 170. The input unit 170 may be provided separately from the photoacoustic apparatus according to the present embodiment.

**Target-Object Information Acquisition Method according to First Embodiment**

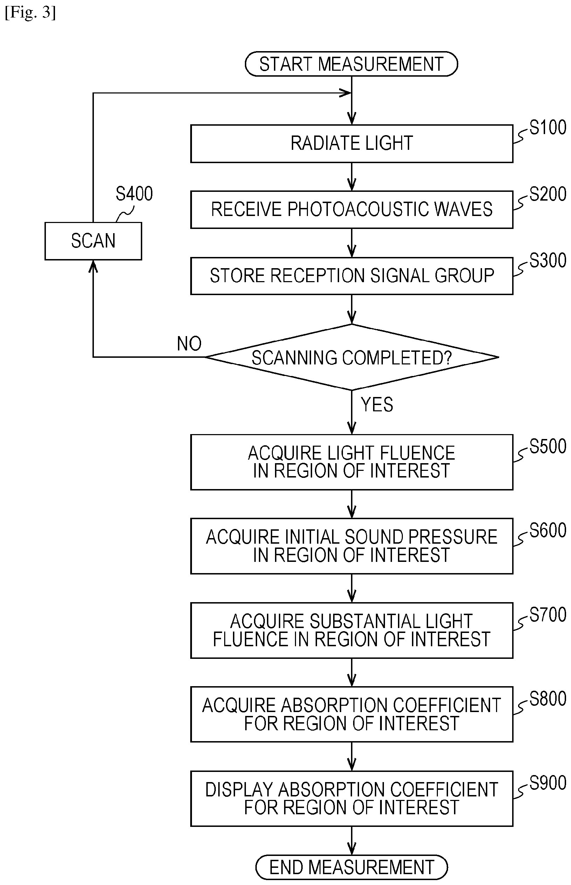

Next, steps of the target-object information acquisition method according to this embodiment will be described with reference to Fig. 3. The steps are executed by the control unit 151 controlling the operation of the respective components of the photoacoustic apparatus.

**S100: Step of Irradiating Inside of Target Object with Light to Generate Photoacoustic Waves**

The light generated by the light source 110 passes through the optical system 120, and the target object 100 is irradiated with the generated light as the pulsed light 121. Then, the pulsed light 121 is absorbed within the target object 100, and photoacoustic waves are produced due to the photoacoustic effect.

**S200: Step of Receiving Photoacoustic Waves and Acquiring Reception Signal Group**

In this step, the probe 130 receives (or detects) the photoacoustic waves, and the transducers 131 output time-series reception signals $p(r_i, t)$, where $r_i$ denotes the position vector of the i-th transducer 131 to be used for imaging, and t denotes time. In this embodiment, the light irradiation timing is set to t = 0.

**S300: Step of Storing Reception Signal Group**

In this step, the time-series reception signal group output from the plurality of transducers 131 is stored in the storage unit 153. The reception signals are stored in association with the scanning positions of the probe 130 and the optical system 120. Preferably, the intensity of the light with which the target object 100 is irradiated is also stored in association with the scanning positions. The use of the intensity of the light with which the target object 100 is irradiated may enable more accurate acquisition of the light fluence in the region of interest in the target object 100 in S500.

**S400: Step of Scanning Probe 130 and Optical System 120**

In this step, the probe 130 and the optical system 120 are scanned and moved to the next positions. After the movement of the probe 130 and the optical system 120, the measurement operation of S100 to S300 is repeatedly performed. If measurement is completed at all the positions, the scanning operation is completed, and the process proceeds to S500.

**S500: Step of Acquiring Light Fluence in Region of Interest**

In this step, the arithmetic unit 152 calculates a light fluence in the region of interest at each scanning position. If the region of interest includes a plurality of minimum units of imaging (such as voxels), a light fluence for each minimum unit, that is, a light fluence distribution in the region of interest, is calculated. The calculation of a light fluence may be based on numerical solution of a transport equation or diffusion equation that describes the behavior of optical energy in a medium that absorbs and scatters light, such as a living body, by the finite element method, the finite difference method, the Monte Carlo method, or the like. If the shape and the like of the target object 100 are simple, a light fluence may be calculated using analytical solution of the transport equation or diffusion equation. The intensity of the illumination light, which is stored in the storage unit 153 in S300, may be loaded into the arithmetic unit 152, and may be used for the calculation of a light fluence to achieve more accurate calculation.

Alternatively, the user may designate information regarding a region in which the target object 100 is irradiated with light, by using the input unit 170 to allow the arithmetic unit 152 to acquire a light fluence in the region of interest. For example, the user may designate the size (such as a spot diameter) of the light radiated from the optical system 120, and the light fluence in a region obtained by extending the designated size of the light in the radiation direction may be set to 1 and to 0 otherwise. Alternatively, the user may directly enter a region in which the target object 100 is irradiated with light, and the light fluence in the designated region may be set to 1 and to 0 otherwise. In the cases described above, the amount of arithmetic required to acquire the light fluence in the region of interest may be reduced. In the cases described above, furthermore, the number of pieces of data to be used to calculate the initial sound pressure in the region of interest in S600, described below, may be reduced.

The transport equation or diffusion equation requires at least two parameters, that is, an absorption coefficient that represents the optical absorption characteristics of the target object and the scattering coefficient that represents the light scattering characteristics of the target object. The above-described parameters may be based on statistic values for the age of the person being examined, a typical value of the living body, which corresponds to the average of the statistic values, or values obtained using an apparatus for measuring the parameters described above, which is different from the apparatus according to this embodiment.

If the light fluence in the region of interest, which is calculated in this step, is less than or equal to a threshold value, the light fluence in the region of interest may be regarded as 0. This may reduce the number of pieces of data to be used to calculate the initial sound pressure in the region of interest in S600, described below. The threshold value may be set in advance or may be entered by the user using the input unit 170.

The acquired light fluence is stored in the storage unit 153 for each scanning position in association with the scanning position.

**S600: Step of Calculating Initial Sound Pressure in Region of Interest**

In this step, the initial sound pressure in the region of interest is calculated. If the region of interest includes a plurality of minimum units of imaging (such as voxels), an initial sound pressure for each minimum unit, that is, the initial sound pressure distribution in the region of interest, is calculated.

For example, an initial sound pressure may be calculated by using a universal backprojection method given by Equation (1).

[Math.1]

$$p_0\left(\mathbf{r}_0\right) = \frac{\sum_i^N b\left(\mathbf{r}_i, t = \frac{\left|\mathbf{r}_i - \mathbf{r}_0\right|}{c}\right) \cdot \Delta\Omega_i}{\sum_i^N \Delta\Omega_i} \quad \cdots (1)$$

$$b\left(\mathbf{r}_i, t\right) = 2p\left(r_i, t\right) - 2t\frac{\partial p\left(r_i, t\right)}{\partial t}$$

where $r_0$ denotes a position vector indicating the position of imaging, $p_0(r_0)$ denotes an initial sound pressure at the position of imaging, and c denotes the sound velocity in the target object 100. In addition, $\Delta\Omega_i$ denotes the solid angle under which the i-th transducer 131 is seen from the position of imaging, and N denotes the number of transducers 131 to be used for imaging.

Equation (1) indicates that time-series reception signals $p(r_i, t)$ are subjected to a process such as differentiation, and are multiplied by the weighting factor of the solid angle, and are subjected to a delay-and-sum. Equation (2), which does not include the differentiation and the weighting of the solid angle in Equation (1), also enables imaging, and the essence of the present invention is not affected if Equation (2) is used. In the following, Equation (2) is used, for convenience of description.

[Math.2]

$$p_0\left(\mathbf{r}_0\right) = \frac{\sum_i^N p\left(\mathbf{r}_i, t = \frac{\left|\mathbf{r}_i - \mathbf{r}_0\right|}{c}\right)}{N} \quad \cdots (2)$$

In this embodiment, a plurality of reception signal groups respectively obtained at a plurality of scanning positions are used to perform imaging. Thus, extending Equation (2) to the case of a plurality of scanning operations yields Equation (3).

[Math.3]

$$p_0(\mathbf{r}_0) = \frac{\sum_{j}^{N_s}\left[\sum_{i}^{N_j} p\left(\mathbf{r}_i^j, t = \frac{|\mathbf{r}_i^j - \mathbf{r}_0|}{c}\right)\right]}{\sum_{j}^{N_s} N_j} \quad \cdots (3)$$

where $\mathbf{r}_i^j$ denotes the position vector of the i-th transducer 131, which is used for imaging, at the j-th scanning position, $N_s$ denotes the number of times scanning is performed, and $N_j$ denotes the number of transducers 131 used for reconstruction in the j-th scanning operation.

Further, Equation (3) is weighted by multiplication by the light fluence for each scanning position, which is acquired in S500, yielding Equation (4).

[Math.4]

$$p_0(\mathbf{r}_0) = \frac{\sum_{j}^{N_s}\left[(N_j \phi_j(\mathbf{r}_0))\sum_{i}^{N_j} p\left(\mathbf{r}_i^j, t = \frac{|\mathbf{r}_i^j - \mathbf{r}_0|}{c}\right)\right]}{\sum_{j}^{N_s}\left[(N_j \phi_j(\mathbf{r}_0))N_j\right]} \quad \cdots (4)$$

In Equation (4), $\varphi_j(\mathbf{r}_0)$ denotes the light fluence at the position of imaging $\mathbf{r}_0$ when light radiation is performed at the j-th scanning position, and $(N_j\varphi_j(\mathbf{r}_0))$ denotes the weighting term based on the light fluence. The reception signal group of the transducers 131, the number of which is equal to $N_j$, is subjected to a delay-and-sum, which is substantially equivalent to the light fluence $\varphi_j(\mathbf{r}_0)$ being radiated $N_j$ times. Thus, $N_j$ is multiplied. Accordingly, weighted reception signals are obtained.

To compensate for a change in the absolute value of a reception signal due to the multiplication of $(N_j\varphi_j(\mathbf{r}_0))$ in the numerator, $(N_j\varphi_j(\mathbf{r}_0))$ is also multiplied in the denominator of Equation (4).

The control unit 151 loads the algorithm of Equation (4), which is stored in the storage unit 153, the reception signal group acquired in S300, and the light fluence acquired in S500 into the arithmetic unit 152, and causes the arithmetic unit 152 to implement the algorithm of Equation (4). Accordingly, the initial sound pressure in the region of interest is obtained, and is stored in the storage unit 153.

A description will now be given of the reduction in artifacts with Equation (4).

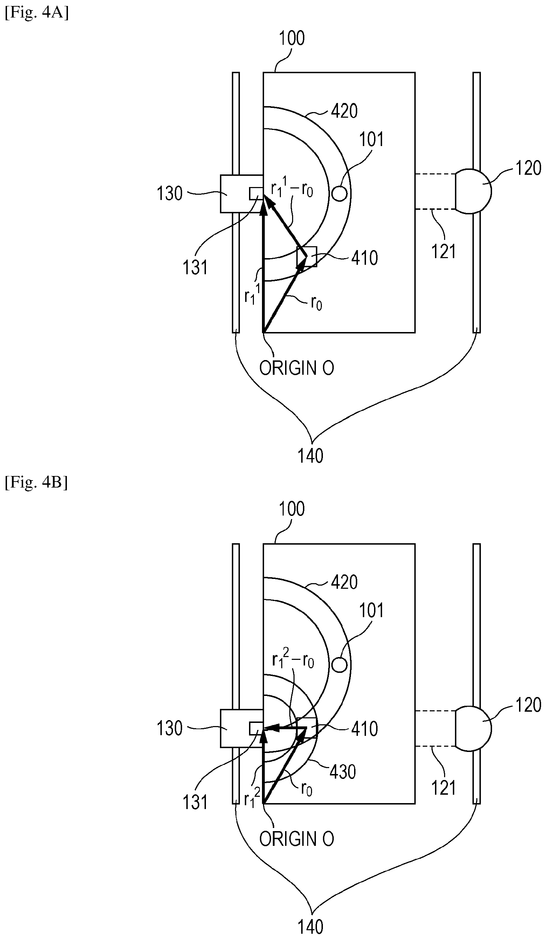

First, the occurrence of artifacts will be described with reference to Figs. 4A and 4B. Figs. 4A and 4B illustrate the scanning of the probe 130 and the optical system 120. In Figs. 4A and 4B, for ease of description, measurement based on scanning is performed at two positions ($N_s$ = 2), and a single transducer 131 ($N_j$ = 1, j = 1, 2) is used.

Consideration is given to the imaging of a region 410 where the light absorber 101 is not present. In this case, the expression in Equation (3) for imaging is modified as shown in Equation (5).

[Math.5]

$$p_0(\mathbf{r}_0) = \frac{\sum_{j}^{N_s}\left[\sum_{i}^{N_j} p\left(\mathbf{r}_i^j, t = \frac{|\mathbf{r}_i^j - \mathbf{r}_0|}{c}\right)\right]}{\sum_{j}^{N_s} N_j} = \frac{p\left(\mathbf{r}_1^1, t = \frac{|\mathbf{r}_1^1 - \mathbf{r}_0|}{c}\right) + p\left(\mathbf{r}_1^2, t = \frac{|\mathbf{r}_1^2 - \mathbf{r}_0|}{c}\right)}{1+1} \quad \cdots (5)$$

Fig. 4A illustrates the (j = 1)-th scanning position, and the first term in the numerator of Equation (5) indicates a reception signal at j = 1. In Fig. 4A, drawing a circular arc 420 with a radius $|r_1^1 - r_0|$ equal to the width of the region 410 and centered at the transducer 131 reveals that the light absorber 101 is included within the circular arc 420. Therefore, time t = |$r_1^1$ - $r_0$|/c of the reception signal includes the amplitude of the initial sound pressure of the light absorber 101. That is, the first term in the numerator of Equation (5) includes the amplitude of the initial sound pressure of the light absorber 101.

Fig. 4B illustrates the (j = 2)-th scanning position, and the second term in the numerator of Equation (5) indicates a reception signal at j = 2. In Fig. 4B, drawing a circular arc 430 in a manner similar to that in the first term in the numerator reveals that the light absorber 101 is not present within the circular arc 430. Therefore, time t = |$r_1^2$-$r_0$|/c of the reception signal does not include the amplitude of the initial sound pressure of the light absorber 101.

As described above, the reception signal at the scanning position j = 2 does not provide the imaging of the light absorber 101 within the region 410 (the second term in the numerator of Equation (5)), whereas the reception signal at the scanning position j = 1 provides the imaging of the region 410 in such a manner that the light absorber 101 is present in the region 410 (the first term in the numerator of Equation (5)). The above-described phenomenon occurs in a region on the circular arc 420, causing artifacts to occur along the circular arc 420.

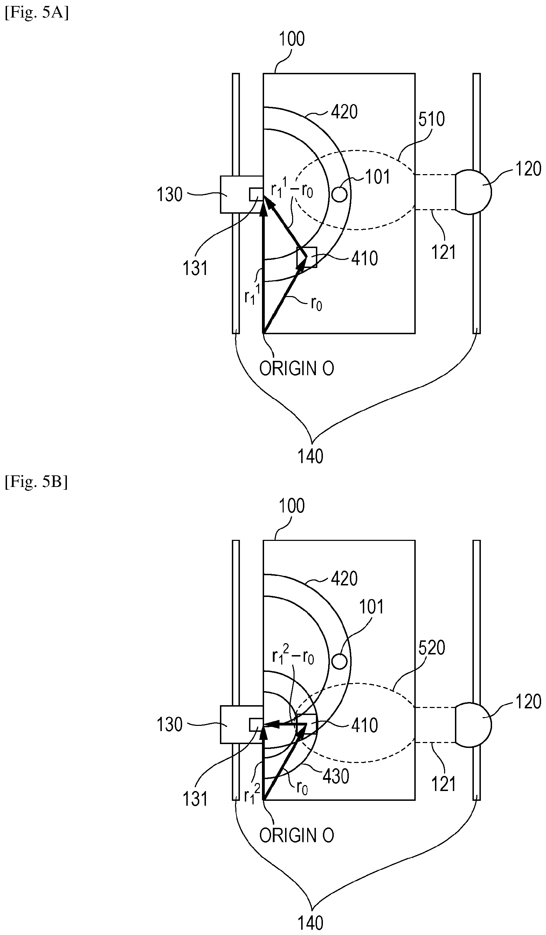

Next, the reduction in artifacts with Equation (4) will be described. Figs. 5A and 5B are diagrams illustrating the light fluences in the target object 100 at the respective scanning positions illustrated in Figs. 4A and 4B. Figs. 5A and 5B illustrate the scanning positions j = 1 and j = 2, respectively, and ranges over which light reaches within the target object 100 are represented by 510 and 520, respectively. Here, the light fluence within the ranges 510 and 520 is set to 1, and the light fluence outside the ranges 510 and 520 is set to 0, for simplicity of description.

Consideration is given to the imaging of the region 410 using the apparatus according to this embodiment. In this case, the expression in Equation (4) for imaging is modified as shown in Equation (6).

[Math.6]

$$p_0(\mathbf{r}_0) = \frac{\displaystyle\sum_j^{N_s}\left[\left(N_j\phi_j(\mathbf{r}_0)\right)\sum_i^{N_j} p\left(\mathbf{r}_i^j, t = \frac{\left|\mathbf{r}_i^j - \mathbf{r}_0\right|}{c}\right)\right]}{\displaystyle\sum_j^{N_s}\left[\left(N_j\phi_j(\mathbf{r}_0)\right)N_j\right]}$$

$$= \frac{1\cdot\phi_1(\mathbf{r}_0)p\left(\mathbf{r}_1^1, t = \frac{\left|\mathbf{r}_1^1 - \mathbf{r}_0\right|}{c}\right) + 1\cdot\phi_2(\mathbf{r}_0)p\left(\mathbf{r}_1^2, t = \frac{\left|\mathbf{r}_1^2 - \mathbf{r}_0\right|}{c}\right)}{\left(1\cdot\phi_1(\mathbf{r}_0)\cdot 1\right) + \left(1\cdot\phi_2(\mathbf{r}_0)\cdot 1\right)} \quad \cdots (6)$$

where $\varphi_1(r_0)$ denotes the light fluence in the region 410 at the scanning position j = 1, and $\varphi_2(r_0)$ denotes the light fluence in the region 410 at the scanning position j = 2. Referring to Fig. 5A, the region 410 is located outside the range 510, and thus $\varphi_1(r_0) = 0$ holds. Referring to Fig. 5B, the region 410 is located within the range 520, and thus $\varphi_2(r_0) = 1$ holds.

Substituting the values given above into Equation (6) yields Equation (7).

[Math.7]

$$p_0(\mathbf{r}_0) = \frac{1 \cdot \phi_1(\mathbf{r}_0) p\left(\mathbf{r}_1^1, t = \dfrac{|\mathbf{r}_1^1 - \mathbf{r}_0|}{c}\right) + 1 \cdot \phi_2(\mathbf{r}_0) p\left(\mathbf{r}_1^2, t = \dfrac{|\mathbf{r}_1^2 - \mathbf{r}_0|}{c}\right)}{\left(1 \cdot \phi_1(\mathbf{r}_0) \cdot 1\right) + \left(1 \cdot \phi_2(\mathbf{r}_0) \cdot 1\right)}$$

$$= \frac{0 \cdot p\left(\mathbf{r}_1^1, t = \dfrac{|\mathbf{r}_1^1 - \mathbf{r}_0|}{c}\right) + 1 \cdot p\left(\mathbf{r}_1^2, t = \dfrac{|\mathbf{r}_1^2 - \mathbf{r}_0|}{c}\right)}{0 + 1} \qquad \cdots (7)$$

$$= p\left(\mathbf{r}_1^2, t = \dfrac{|\mathbf{r}_1^2 - \mathbf{r}_0|}{c}\right)$$

Equation (7) reveals that weighting the reception signal by the light fluence in the imaging region allows the $p(r_1^1, t=|r_1^1-r_0|/c)$ term, which may cause the occurrence of an artifact, to fall away.

In actuality, the light fluence in the target object 100 may not necessarily be equal to 0 or 1, and the light has an intensity distribution. The intensity of the light with which the target object 100 is irradiated exponentially decays as the distance from the irradiation position increases, due to the optical absorption characteristics and light scattering characteristics of the target object 100. The region 410 and the irradiation position of light are far from each other at the scanning position j = 1, and are close to each other at the scanning position j = 2. Accordingly, $\varphi_1(r_0) < \varphi_2(r_0)$ is satisfied. Thus, in Equation (6), the $p(r_1^1, t=|r_1^1-r_0|/c)$ term, which may cause the occurrence of an artifact, contributes a small amount, resulting in the effect of reducing artifacts being actually achieved. In a case where the light fluence is binarized, such as 0 and 1, or is converted to multilevel values using a step table, it may be sufficient that an arbitrary threshold value is determined and is applied to the light fluence based on the intensity distribution.

For a signal group received when the light fluence in the region of interest is equal to 0, the initial sound pressure in the region of interest can be obtained by using a light fluence weight of 0, that is, without using the signal group. This reduces the number of pieces of data to be used to obtain the initial sound pressure in the region of interest, enabling rapid acquisition of the initial sound pressure.

For a signal group received when the light fluence in the region of interest is lower than the threshold value, the initial sound pressure in the region of interest may be obtained without using the signal group. This also reduces the number of pieces of data to be used to obtain the initial sound pressure in the region of interest, enabling rapid acquisition of the initial sound pressure.

In this embodiment, the light fluence itself is used as a weighting factor. Any other weighting factor based on the light fluence may be used to achieve advantages. For example, a value proportional to the light fluence may be used as a weighting factor. Alternatively, for example, in a case where a non-linear photoacoustic wave proportional to the power of the light fluence is detected to obtain target-object information, the power of the light fluence or a value proportional to the power of the light fluence may be used as a weighting factor.

The foregoing description is also applicable to the case where a plurality of transducers 131 is used and the number of times scanning is performed is greater than or equal to three. The essence of the present invention is not affected by changing the number of transducers 131 and the number of times scanning is performed.

**S700: Step of Acquiring Substantial Light Fluence in Region of Interest**

In this step, a substantial light fluence in the region of interest is obtained. The substantial light fluence in the region of interest is necessary to obtain an absorption coefficient that is target-object information on the region of interest in S900.

An absorption coefficient $\mu(r_0)$ and an initial sound pressure $p(r_0)$ have a relationship of Equation (8).
[Math. 8]

$$p_0(\mathbf{r}_0) = \Gamma \cdot \mu(\mathbf{r}_0) \cdot \Phi(\mathbf{r}_0) \quad \cdots (8)$$

where $\Gamma$ denotes the so-called Gruneisen coefficient, which is obtained by dividing the product of a volume expansion coefficient of the target object 100 and the square of the sound velocity by a specific heat at constant pressure. It is known that the Gruneisen coefficient has a substantially constant specified value for a living body or the like. In addition, $\Phi(r_0)$ denotes the average light fluence at the position $r_0$. In this embodiment, the average light fluence is a value obtained by averaging the light fluences in the region of interest at the respective scanning positions. According to Equation (8), the initial sound pressure is divided by the average light fluence and the Gruneisen coefficient to obtain the absorption coefficient $\mu_c(r_0)$ for the region of interest.

The average light fluence $\Phi(r_0)$ in which the light fluence in the region of interest at each scanning position is taken into account is expressed by Equation (9).

[Math.9]

$$\Phi(\mathbf{r}_0) = \frac{\sum_{j}^{N_s}\left(N_j \phi_j(\mathbf{r}_0)\right)}{\sum_{j}^{N_s} N_j} \quad \cdots (9)$$

As described above, a group of $N_j$ signals is subjected to a delay-and-sum to calculate the initial sound pressure, which is substantially equivalent to light being applied $N_j$ times at each scanning position. In Equation (9), accordingly, the light fluence $\varphi_j(r_0)$ at each scanning position is multiplied by $N_j$.

In this embodiment, the light fluence is weighted for the calculation of the initial sound pressure in S600. That is, the substantial light fluence at each scanning position is equal to a light fluence obtained by weighting the light fluence $(N_j\varphi_j(r_0))$ by the same light fluence. Thus, in this embodiment, the average light fluence $\Phi'(r_0)$ is expressed by Equation (10).

[Math. 10]

$$\Phi'(\mathbf{r}_0) = \frac{\sum_{j}^{N_s}\left[\left(N_j \phi_j(\mathbf{r}_0)\right) \cdot \left(N_j \phi_j(\mathbf{r}_0)\right)\right]}{\sum_{j}^{N_s}\left[\left(N_j \phi_j(\mathbf{r}_0)\right) \cdot N_j\right]} \quad \cdots (10)$$

In the numerator of Equation (10), the light fluence is weighted by the same light fluence. Thus, to compensate for a change in the absolute value due to the weighting, the same light fluence $(N_j\varphi_j(r_0))$ is also multiplied in the denominator.

Equation (10) indicates a substantial average light fluence that contributes to the initial sound pressure obtained by weighting a reception signal group by a light fluence. The substantial average light fluence is referred to as the substantial light fluence.

The control unit 151 loads the algorithm of Equation (10), which is stored in the storage unit 153, and the light fluence acquired in S500 into the arithmetic unit 152, and causes the arithmetic unit 152 to implement the algorithm of Equation (10). Accordingly, the substantial light fluence $\Phi'(r_0)$ in the region of interest is obtained in the way described above, and is stored in the storage unit 153.

**S800: Step of Acquiring Absorption Coefficient for Region of Interest**

In this step, an absorption coefficient for the region of interest is obtained through correction using the initial sound pressure in the region of interest, which is acquired in S600, and the substantial light fluence in the region of interest, which is acquired in S700.

The control unit 151 loads the initial sound pressure $p(r_0)$ acquired in S600, which is stored in the storage unit 153, and the substantial light fluence $\Phi(r_0)$ acquired in S700 into the arithmetic unit 152, and causes the arithmetic unit 152 to calculate an absorption coefficient $\mu_c(r_0)$ for the region of interest in accordance with Equation (8). Through the correction described above, an absorption coefficient $\mu_c(r_0)$ for the region of interest is obtained, and is stored in the storage unit 153.

**S900: Step of Displaying Absorption Coefficient for Region of Interest**

In this step, the absorption coefficient for the region of interest, which is acquired in S800, is displayed on the display unit 160. The arithmetic unit 152 performs a process, such as brightness conversion, on the absorption coefficient for the region of interest so that the absorption coefficient is displayed on the display unit 160, to generate image data of the absorption coefficient, and outputs the image data to the display unit 160. The absorption coefficient displayed on the display unit 160 is information with high quantitativity and suppressed occurrence of artifacts, and is thus information suitable for an operator such as a physician to perform diagnosis or the like. The arithmetic unit 152 may cause a value of target-object information on a specific position in the target object 100 to be displayed on the display unit 160.

In this embodiment, an absorption coefficient is obtained as target-object information, by way of example. Alternatively, the initial sound pressure acquired in S600 may be obtained as target-object information, and may be displayed in S900. Instead of this, target-object information for a plurality of wavelengths may be obtained and displayed. Alternatively, absorption coefficients corresponding to a plurality of wavelengths may be used to obtain the concentration of the substances in the target object, and the resulting concentration may be displayed as target-object information.

In this embodiment, target-object information is obtained after all the scanning (measurement) operations are completed. Alternatively, target-object information may be obtained while a scanning operation is performed. For example, the following process may be performed: Immediately after photoacoustic waves are received in S200, the numerators of Equation (4) and Equation (10) are computed and the denominators of Equation (4) and Equation (10) are computed. Then, the results of the computation of the numerators and the denominators are separately summed up in the storage unit 153. The term "summing up" refers to adding together the results of computation for each scanning position.

When all the scanning operations are completed, the processing of S500 is omitted, and, in S600, the summed-up value of the numerator of Equation (4) is divided by the summed-up value of the denominator to obtain an initial sound pressure. In S800, the summed-up value of the numerator of Equation (10) is divided by the summed-up value of the denominator to obtain a substantial light fluence. Performing scanning and computation in parallel may reduce the time taken to obtain target-object information. In addition, not all the reception signals need to be stored in the storage unit 153, resulting in a reduction in the memory area.

In addition, in a case where an absorption coefficient is obtained as target-object information, the summing up operation described above may be performed only on the numerators of Equation (4) and Equation (10) because the denominators of Equation (4) and Equation (10) are identical. That is, the processing of S600 and S700 is omitted, and, in S800, the summed-up value of the numerator of Equation (4) is divided by the summed-up value of the numerator of Equation (10) (and the Gruneisen coefficient $\Gamma$) to obtain an absorption coefficient.

In this embodiment, a reception signal group is weighted by the light fluence for every scanning position to obtain target-object information. Alternatively, all the scanning operations may be divided into a plurality of groups, and each group may be regarded as a single scanning operation. That is, it is assumed that irradiation with light is simultaneously applied at respective scanning positions within each group, and photoacoustic waves are received by a virtual probe obtained by combining the probes 130 at respective scanning positions within the same group.

Specifically, the pulsed light beams 121 radiated at respective scanning positions within a group are regarded as being radiated simultaneously, and the light fluence in the region of interest is calculated. An initial sound pressure is calculated using the reception signals of all the transducers 131 included in the virtual probe. The light fluence for each group, which is obtained in the way described above, is weighted by a reception signal group for the corresponding group, and each group is regarded as a single scanning operation to implement Equation (4).

For example, consideration is given to the case where, in Figs. 4A and 4B, the probe 130 and the optical system 120 can also be scanned in the direction perpendicular to the page of the drawing. In this case, scanning operations in the direction parallel to the page of the drawing are regarded as a single group, and Equation (4) is implemented for the scanning operations in the direction perpendicular to the page of the drawing. As described above, the amount of arithmetic in the arithmetic unit 152 is reduced while the effect of reducing artifacts is achieved, resulting in a reduction in the time taken to acquire target-object information.

In the target-object information acquisition method according to this embodiment, accordingly, it may be possible to obtain target-object information with high quantitativity and reduced artifacts.

**Second Embodiment**

A photoacoustic apparatus according to a second embodiment will be described. The photoacoustic apparatus includes a probe having transducers for receiving photoacoustic waves such that the transducers are located on a spherical surface. In the second embodiment, the same or similar components as or to those in the first embodiment are basically given the same numerals and are not described herein.

**Configuration of Photoacoustic Apparatus according to Second Embodiment**

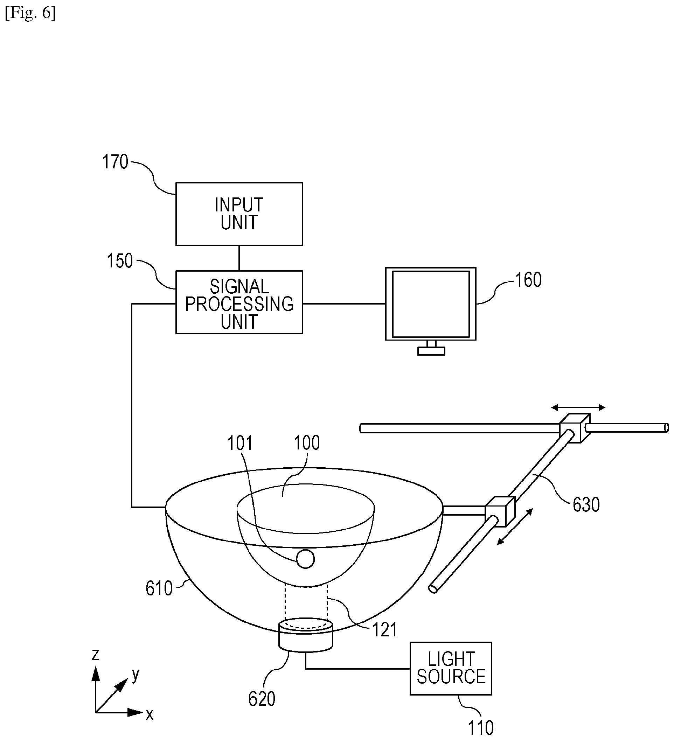

Fig. 6 is a schematic diagram of the photoacoustic apparatus according to this embodiment. A description will now be given of each of the components of the photoacoustic apparatus. The photoacoustic apparatus includes the light source 110, an optical system 620, a probe 610 having a plurality of transducers 611, a scanning unit 630, the signal processing unit 150, and the display unit 160. The target object 100 is a target of measurement. The scanning unit 140 scans the probe 610 and the optical system 620 over the target object 100.

**Optical System 620**

The optical system 620 according to this embodiment is integrally formed with the probe 610. When the probe 610 is scanned by the scanning unit 140, the optical system 620 is also scanned over the target object 100.

**Probe 610**

In this embodiment, the probe 610 has a spherical shape. During measurement, as illustrated in Fig. 6, the probe 610 mounts the target object 100 in it, and receives photoacoustic waves.

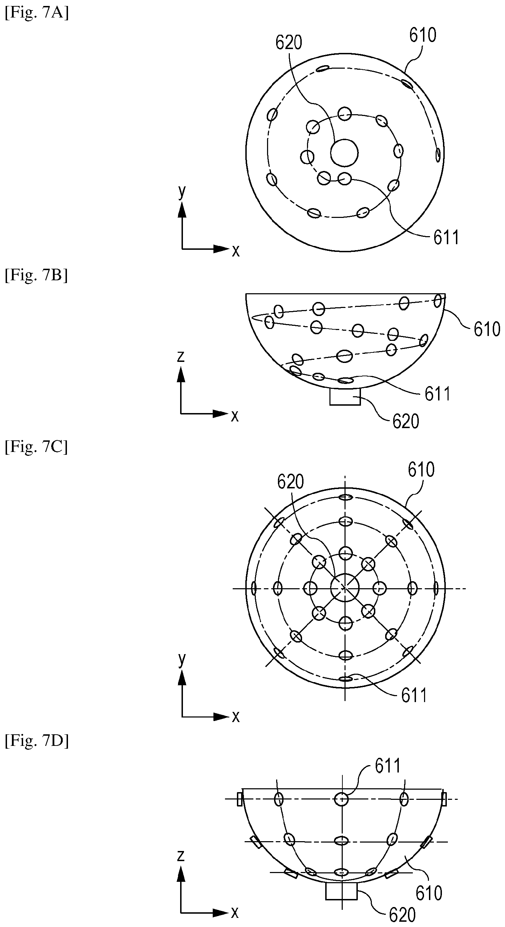

Figs. 7A to 7D are diagrams illustrating the details of the probe 610. Figs. 7A and 7B illustrate the probe 610 in which the transducers 611 are arranged in a spiral pattern. Figs. 7C and 7D illustrate the probe 610 in which the transducers 611 are radially arranged. Figs. 7A and 7C are diagrams of the probe 610, viewed in the z-direction in Fig. 6, and Figs. 7B and 7D are diagrams of the probe 610, viewed in the y-direction in Fig. 6. In either case, the transducers 611 are arranged on a spherical surface so as to cover the probe 610. The transducers 611 are configured to receive photoacoustic waves generated by the target object 100 at various angles, resulting in a larger amount of information being obtained than that in a planar probe. In particular, a large amount of information on photoacoustic waves generated near the center of curvature of the probe 610 formed into a spherical surface may be obtained. In a case where pulsed light is emitted toward the center of curvature of the probe 610, the sound pressure of a photoacoustic wave generated around the center of curvature relatively increases. In this case, a portion near the center of curvature is preferably weighted a large weighting factor for reconstruction because a region where the generated sound pressure of the photoacoustic wave is high overlaps a region where a generated photoacoustic wave is received with high sensitivity.

In Figs. 7A to 7D, the transducers 611 are arranged spirally or radially. However, the arrangement of the transducers 611 is not limited to this pattern. For example, the transducers 611 may be arranged on a spherical surface in a lattice pattern.

The space between the probe 610 and the target object 100 is filled with a medium that allows photoacoustic waves to propagate. Preferably, the medium, as well as allowing photoacoustic waves to propagate, provides matching of acoustic characteristics on the interface between the medium and the target object 100 or the transducers 611 and has as high a photoacoustic wave transmittance as possible.

**Scanning Unit 630**

The scanning unit 630 relatively scans the probe 610 and the optical system 620 over the target object 100. In Fig. 6, the scanning unit 630 scans the probe 610 and the optical system 620 over the x-y plane.

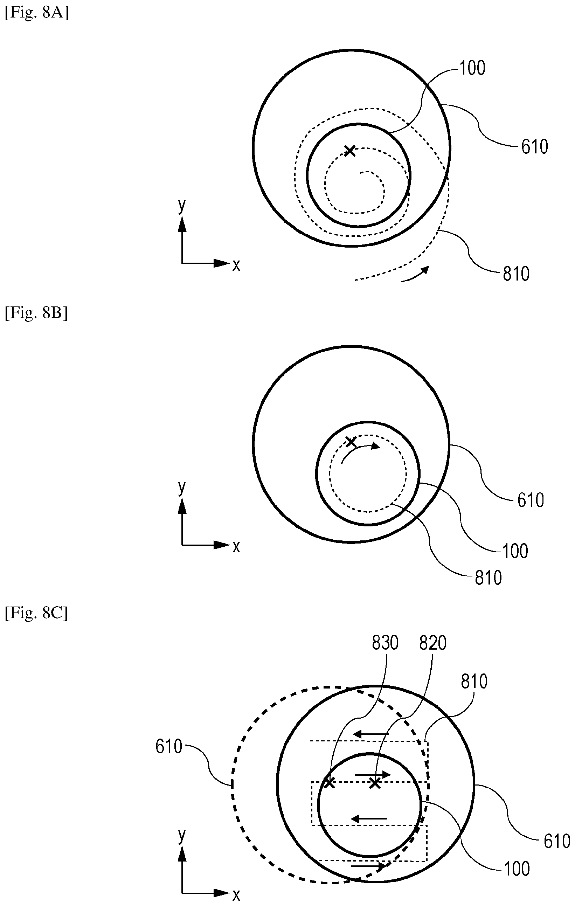

Figs. 8A to 8C are diagrams illustrating the scanning of the probe 610 over the x-y plane, viewed in the z-direction. The probe 610 is scanned within the x-y plane along a locus 810 by the scanning unit 630. Figs. 8A to 8C are diagrams illustrating the probe 610 when the center of curvature of the probe 610 is positioned at cross-mark (x). The locus 810 may be in the shape of, for example, spiral in Fig. 8A, circular in Fig. 8B, straight line in Fig. 8C, or the like. The solid-line shown in Fig. 8C illustrates the probe 610 when the center of curvature of the probe 610 is positioned at a cross-mark 820. The dot-line shown in Fig. 8C illustrates the probe 610 when the center of curvature of the probe 610 is positioned at a cross-mark 830. The shape of the locus 810 is not limited to those described above, and the locus 810 may be in any shape.

In the manner described above, scanning the probe 610 over the target object 100 enables photoacoustic waves to be received at more positions at more angles, increasing the amount of information to obtain target-object information and improving the accuracy of the target-object information. In particular, resolution, contrast, and so forth may be improved in the imaging of the target-object information. In addition, the probe 610 may be scanned in the z-direction in Fig. 6 to improve image quality.

**Target-Object Information Acquisition Method according to Second Embodiment**

Similarly to the first embodiment, the photoacoustic apparatus according to this embodiment acquires target-object information through the steps illustrated in Fig. 3. The steps are implemented by the control unit 151 controlling the operation of the respective components of the photoacoustic apparatus. The steps are substantially the same as those described in the first embodiment, and are not described herein.

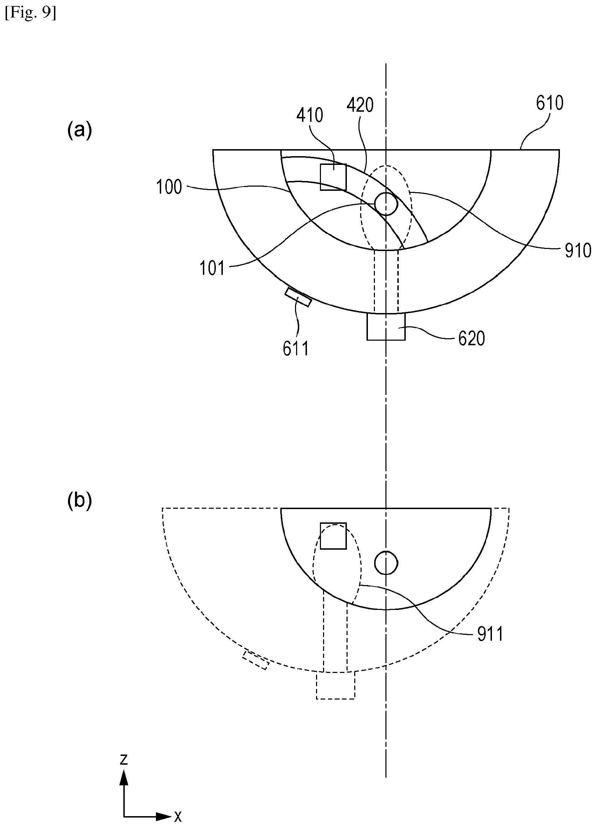

The reduction in artifacts in the imaging of target-object information according to this embodiment will be described with reference to Fig. 9, illustrating the scanning in Fig. 8C, viewed in the y-direction.

Fig. 9 is a diagram of the probe 610 when the center of curvature of the probe 610 is positioned at each of the cross-marks 820 and 830 in Fig. 8C, viewed in the y-direction, respectively. Ranges 910 and 911 represent regions over which light radiated at the respective scanning positions reaches within the target object 100. The term "range over which light reaches" is defined as in the first embodiment described above.

In part (a) of Fig. 9, the region 410 and the circular arc 420 are the same as those described above in the first embodiment. The circular arc 420 is related to the transducer 611 illustrated in part (a) of Fig. 9. As in the first embodiment, the region 410 is included within the circular arc 420. Thus, an artifact of the light absorber 101 occurs in the region 410 according to Equation (3), which is an equation for imaging for an existing photoacoustic apparatus. The photoacoustic apparatus according to this embodiment uses Equation (4) for imaging, and the region 410 is located outside the range 910 over which light reaches. Thus, a reception signal of the transducer 611, which includes a signal of the light absorber 101, is weighted by the light fluence which has a small value.

In part (b) of Fig. 9, in contrast, the region 410 is located within the range 911. Thus, a reception signal of the transducer 611, which does not include a signal of the light absorber 101, is weighted by the light fluence which has a large value. Accordingly, the reception signal in part (b) of Fig. 9 largely contributes to the imaging of the region 410, resulting in a reduction in artifacts caused by the light absorber 101.

In the second embodiment, a photoacoustic apparatus is configured such that the probe 610, which has a different shape from that in the first embodiment, is scanned using a different scanning method. Such a photoacoustic apparatus may also reduce artifacts by using the target-object information acquisition method according to the embodiments of the present invention, which is given by the expression in Equation (4) for imaging. That is, the target-object information acquisition method according to the embodiments of the present invention is applicable regardless of the layout of transducers or the scanning method so long as light is radiated under different conditions by, for example, scanning an optical system.

**Example**

In the following, the results of the simulation of the target-object information acquisition method according to the first or second embodiment will be described.

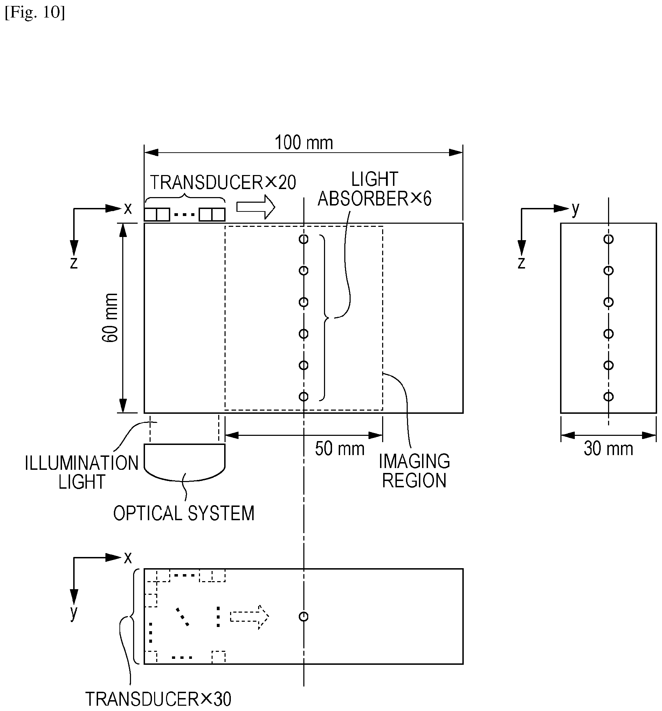

The use of a computational model (numerical phantom) as illustrated in Fig. 10 is assumed.

The size of the numerical phantom is 100 mm, 30 mm, and 60 mm in the x, y, and z directions, respectively. The optical characteristic values of the numerical phantom are an absorption coefficient $\mu_a$ of 0.005 per mm, which is as large as an absorption coefficient of a living body, a scattering coefficient $\mu_s'$ of 1 per mm, which is as large as a scattering coefficient a living body, and a Gruneisen coefficient $\Gamma$ of 1. The numerical phantom contains six light absorbers each having a diameter of 1 mm and an absorption coefficient $\mu_a$ of 0.05 per mm. The light absorbers are located at positions of x = 50 mm and y = 15 mm and at positions of z = 5, 15, 25, 35, 45, and 55 mm.

Transducers, each formed into a rectangular shape having sides of 1 mm each, are arranged in 20 columns in the x-direction and 30 rows in the y-direction, and constitute a 600-element probe. The probe scans over the x-y plane at intervals of 1 mm in the x-direction. An optical system, which serves to radiate light, radiates light over a range of 20 mm and 30 mm in the x-direction and y-direction, respectively. The optical system is scanned synchronously with the probe at intervals of 1 mm. Scanning is performed up to a boundary of the numerical phantom at x = 100 mm.

First, in the numerical phantom illustrated in Fig. 10, a light fluence distribution in the numerical phantom is computed at each scanning position. A light fluence distribution was computed by solving a diffusion equation by the finite element method by using illumination light at each scanning position as a light source. A light fluence at each light absorber position is multiplied by an absorption coefficient of a light absorber to obtain an initial sound pressure of a photoacoustic wave. A photoacoustic wave to be detected by each transducer is computed using an initial sound pressure and analytical solution of a wave equation that describes the propagation of the photoacoustic wave to obtain a reception signal. The operation described above is performed for all the scanning positions.

Next, the light fluence distributions obtained at the respective scanning positions and reception signals are applied to Equation (4) to compute an initial sound pressure distribution in the numerical phantom. Further, the light fluence distributions at the respective scanning positions are applied to Equation (10) to compute a substantial light fluence distribution.

The initial sound pressure distribution is divided by the substantial light fluence distribution to image the absorption coefficient distribution. Imaging was performed on an imaging region (50 mm in the x-direction and 60 mm in the z-direction) illustrated in Fig. 10.

For the purpose of comparison, an image of an absorption coefficient distribution was also computed by using Equation (3), which is an existing equation for imaging.

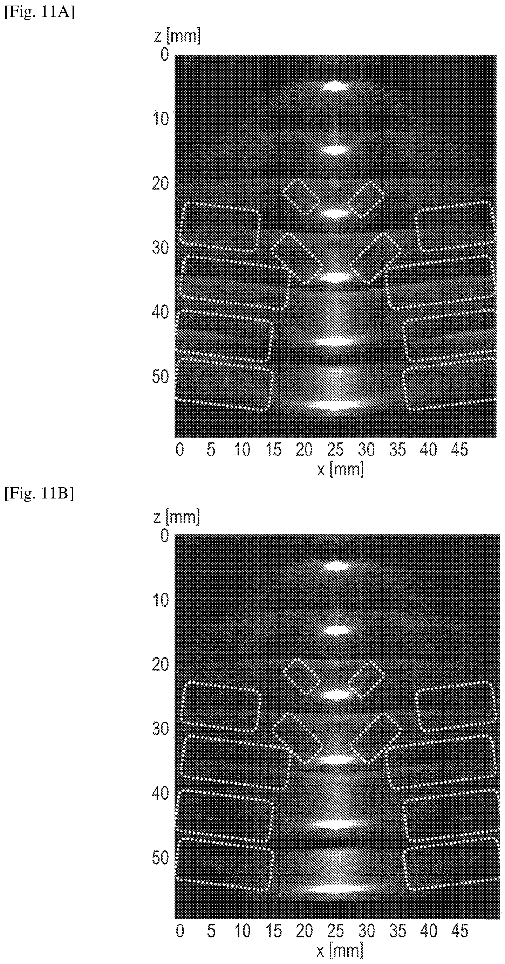

Figs. 11A and 11B illustrate the absorption coefficient distribution images obtained as a result of the computations described above. Figs. 11A and 11B illustrate images obtained by projecting the maximum value in the y-direction onto the x-z plane (Maximum Intensity Projection (MIP)). Fig. 11A illustrates the existing image based on Equation (3), and Fig. 11B illustrates the image of the present invention based on Equation (4). Figs. 11A and 11B reveal that artifacts in regions indicated by broken lines, which are visible in Fig. 11A, are reduced in the corresponding regions in the image of the present invention illustrated in Fig. 11B.

As described above, an embodiment of the present invention may provide a photoacoustic apparatus that enables a reduction in the occurrence of artifacts. Another embodiment of the present invention may provide a photoacoustic apparatus capable of acquiring target-object information with high quantitativity and reduced artifacts.

### Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

### Claims

1. A photoacoustic apparatus comprising:

   an irradiation unit (110, 120) configured to irradiate a target object (100) with light;
   a probe (130) including a plurality of transducers (131) and configured to receive acoustic waves generated from the target object irradiated with the light and to output a reception signal group;
   a storage unit (153) configured to store the reception signal group output from the plurality of transducers; and
   a processing unit (150) configured to determine target-object information in a region of interest of the target object,
   wherein the irradiation unit is configured to irradiate the target object with the light at a different position at each of a plurality of timings,
   wherein the probe is configured to receive, for each of the plurality of timings an acoustic wave generated by irradiated the target object with the light, and to output a plurality of reception signal groups respectively corresponding to the plurality of timings,
   wherein the storage unit is configured to store the plurality of reception signal groups, and
   wherein the processing unit is further configured to
   determine for each of the plurality of timings, a light fluence in the region of interest,
   obtain, for each of the plurality of timings, reception signals corresponding to the acoustic wave generated from the region of interest and received by the plurality of transducers, from the plurality of reception signal groups stored in the storage unit,
   weight, for each of the plurality of timings, the reception signals corresponding to the acoustic wave generated from the region of interest and received by the plurality of transducers with a weighting factor, and
   determine the target-object information in the region of interest based on the plurality of weighted reception signals respectively corresponding to the plurality of timings; and **characterized in that**
   the processing unit is configured to determine, for each of the plurality of timings, the weighting factor based on the light fluence in the region of interest.

2. The photoacoustic apparatus according to Claim 1, wherein the processing unit (150) is further configured to determine an initial sound pressure, for each of the plurality of timings, based on the plurality of weighted reception signals,

weight the light fluence in the region of interest with the weighting factor for each of the plurality of timings to calculate a weighted light fluence for each of the plurality of timings, and

determine the target-object information based on the initial sound pressure and the weighted light fluence for each of the plurality of timings.

3. The photoacoustic apparatus according to Claim 1 or 2, wherein the weighting factor is the light fluence in the region of interest.

4. The photoacoustic apparatus according to any one of Claims 1 to 3, wherein the weighting factor is 0 in a case where the light fluence in the region of interest is lower than a threshold value.

5. The photoacoustic apparatus according to any one of Claims 1 to 4,

wherein the processing unit (150) is further configured to obtain information regarding a region in which the target object (100) is irradiated with light designated by a user, and

determine the light fluence in the region of interest, based on the information regarding the region in which the target object (100) is irradiated with light.

6. The photoacoustic apparatus according to Claim 5,

wherein the information regarding the region is information on a size of the light emitted from the irradiation unit (110, 120), and

wherein the processing unit (150) is configured to determine the light fluence in the region of interest based on the information on the size of the light by determining an extended region by extending a region having the size of the light in a radiation direction and determining that the light fluence inside the extended region is 1 and the light fluence outside the extended region is 0.

7. The photoacoustic apparatus according to any one of Claims 1 to 5, wherein the processing unit (150) is further configured to obtain a light fluence in the region of interest, by using a transport equation or diffusion equation based on optical absorption characteristics and light scattering characteristics of the target object (100).

8. The photoacoustic apparatus according to any one of Claims 1 to 7, wherein the probe (130) is formed to have a spherical surface, and the plurality of transducers (131) are located on the spherical surface.

9. The photoacoustic apparatus according to any one of Claims 1 to 8, wherein the processing unit is further configured to cause a display unit (160) to display the target-object information.

10. The photoacoustic apparatus according to any one of Claims 1 to 9, wherein the irradiation unit includes a light source (110), an optical system (120) configured to guide light from the light source into the target object (100), and a movement unit (140) configured to move the optical system (120) with respect to the target object (100).

11. The photoacoustic apparatus according to Claim 10, wherein the movement unit (140) is configured to move the optical system (120) and the probe (130) with respect to the target object (100) while maintaining a positional relationship between the optical system and the probe.

12. A signal processing method for determining target-object information in a region of interest of a target object, based on a plurality of reception signal groups respectively corresponding to a plurality of timings, the plurality of reception signal groups being output from a plurality of transducers in response to receipt of acoustic waves generated by irradiating the target object with light at a different position at each of the plurality of timings, the signal processing method comprising:

determining, for each of the plurality of timings, a light fluence in the region of interest;
obtaining, for each of the plurality of timings, reception signals corresponding to the acoustic wave generated from the region of interest and received by the plurality of transducers, from the plurality of reception signals,
weighting, for each of the plurality of timings, the reception signals corresponding to the acoustic wave generated from the region of interest and received by the plurality of transducers with a weighting factor; and
determining the target-object information in the region of interest based on the plurality of weighted reception signals respectively corresponding to the plurality of timings; and **characterized by**

determining, for each of the plurality of timings, the weighting factor based on the light fluence in the region of interest.

13. A computer program comprising instructions which, when the program is executed by a computer, cause the computer to perform the method of Claim 12.

**Patentansprüche**

1. Photoakustische Vorrichtung, umfassend:

eine Bestrahlungseinheit (110, 120), die konfiguriert ist, ein Zielobjekt (100) mit Licht zu bestrahlen;
eine Sonde (130) mit mehreren Wandlern (131), die konfiguriert ist, vom mit dem Licht bestrahlten Zielobjekt erzeugte akustische Wellen zu empfangen und eine Empfangssignalgruppe auszugeben;
eine Speichereinheit (153), die konfiguriert ist, die von den mehreren Wandlern ausgegebene Empfangssignalgruppe zu speichern; und
eine Verarbeitungseinheit (150), die konfiguriert ist, Zielobjektinformation in einem Betrachtungsgebiet des Zielobjekts zu bestimmen,
wobei die Bestrahlungseinheit konfiguriert ist, das Zielobjekt zu mehreren Zeitpunkten jeweils mit dem an einer anderen Position befindlichen Licht zu bestrahlen,
wobei die Sonde konfiguriert ist, für die mehreren Zeitpunkte jeweils eine akustische Welle zu empfangen, die durch Bestrahlen des Zielobjekts mit dem Licht erzeugt wurde, und mehrere Empfangssignalgruppen auszugeben, die jeweils den mehreren Zeitpunkten entsprechen,
wobei die Speichereinheit konfiguriert ist, die mehreren Empfangssignalgruppen zu speichern, und
wobei die Verarbeitungseinheit ferner konfiguriert ist,
für die mehreren Zeitpunkte jeweils eine Leuchtstärke im Betrachtungsgebiet zu bestimmen,
für die mehreren Zeitpunkte jeweils Empfangssignale, die der aus dem Betrachtungsgebiet erzeugten und durch die mehreren Wandler empfangenen akustischen Welle entsprechen, von den mehreren in der Speichereinheit gespeicherten Empfangssignalgruppen zu erfassen,
für die mehreren Zeitpunkte jeweils die Empfangssignale, die der aus dem Betrachtungsgebiet erzeugten und durch die mehreren Wandler empfangenen akustischen Welle entsprechen, mit einem Gewichtungsfaktor zu gewichten, und
die Zielobjektinformation im Betrachtungsgebiet basierend auf den mehreren gewichteten Empfangssignalen zu bestimmen, welche jeweils den mehreren Zeitpunkten entsprechen; und **dadurch gekennzeichnet, dass** die Verarbeitungseinheit konfiguriert ist, für die mehreren Zeitpunkte jeweils den Gewichtungsfaktor basierend auf der Lichtstärke im Betrachtungsgebiet zu bestimmen.

2. Photoakustische Vorrichtung nach Anspruch 1, wobei die Verarbeitungseinheit (150) ferner konfiguriert ist,

einen Anfangsschalldruck basierend auf den mehreren gewichteten Empfangssignalen für einen jeweiligen der mehreren Zeitpunkte zu bestimmen,
die Lichtstärke im Betrachtungsgebiet für einen jeweiligen der mehreren Zeitpunkte mit dem Gewichtungsfaktor zu gewichten, um für einen jeweiligen der mehreren Zeitpunkte eine gewichtete Lichtstärke zu berechnen, und
Zielobjektinformation basierend auf dem Anfangsschalldruck und der gewichteten Lichtstärke für einen jeweiligen der mehreren Zeitpunkte zu bestimmen.

3. Photoakustische Vorrichtung nach Anspruch 1 oder 2, wobei der Gewichtungsfaktor die Lichtstärke im Betrachtungsgebiet ist.

4. Photoakustische Vorrichtung nach einem der Ansprüche 1 bis 3, wobei der Gewichtungsfaktor 0 ist, falls die Lichtstärke im Betrachtungsgebiet niedriger als ein Schwellenwert ist.

5. Photoakustische Vorrichtung nach einem der Ansprüche 1 bis 4,

wobei die Verarbeitungseinheit (150) ferner konfiguriert ist, Information über ein durch einen Benutzer bestimmtes Gebiet zu erfassen, in dem das Zielobjekt (100) mit Licht bestrahlt wird, und
basierend auf der Information über das Gebiet, in dem das Zielobjekt (100) mit Licht bestrahlt wird, die Lichtstärke im Betrachtungsgebiet zu bestimmen.

**6.** Photoakustische Vorrichtung nach Anspruch 5, wobei die Information über das Gebiet Information über eine Größe des von der Bestrahlungseinheit (110, 120) emittierten Lichts ist, und

wobei die Verarbeitungseinheit (150) konfiguriert ist, die Lichtstärke im Betrachtungsgebiet basierend auf der Information über die Größe des Lichts zu bestimmen, indem ein erweitertes Gebiet bestimmt wird durch Erweitern eines Gebiets mit der Größe des Lichts in einer Bestrahlungsrichtung und Bestimmen, dass die Lichtstärke innerhalb des erweiterten Gebiets 1 ist und die Lichtstärke außerhalb des erweiterten Gebiets 0 ist.

**7.** Photoakustische Vorrichtung nach einem der Ansprüche 1 bis 5, wobei die Verarbeitungseinheit (150) ferner konfiguriert ist, eine Lichtstärke im Betrachtungsgebiet zu erfassen, indem eine auf optischen Absorptionseigenschaften und Lichtstreuungseigenschaften des Zielobjekts (100) basierende Transportgleichung oder Diffusionsgleichung verwendet wird.

**8.** Photoakustische Vorrichtung nach einem der Ansprüche 1 bis 7, wobei die Sonde (130) so geformt ist, dass sie eine kugelförmig gewölbte Oberfläche hat, und die mehreren Wandler (131) auf der kugelförmig gewölbten Oberfläche angeordnet sind.

**9.** Photoakustische Vorrichtung nach einem der Ansprüche 1 bis 8, wobei die Verarbeitungseinheit ferner konfiguriert ist, eine Anzeigeeinheit (160) zum Anzeigen der Zielobjektinformation zu veranlassen.

**10.** Photoakustische Vorrichtung nach einem der Ansprüche 1 bis 9, wobei die Bestrahlungseinheit umfasst: eine Lichtquelle (110), ein optisches System, das konfiguriert ist, Licht von der Lichtquelle in das Zielobjekt (100) zu leiten, und eine Bewegungseinheit (140), die konfiguriert ist, das optische System (120) bezüglich des Zielobjekts (100) zu bewegen.

**11.** Photoakustische Vorrichtung nach Anspruch 10, wobei die Bewegungseinheit (140) konfiguriert ist, das optische System (120) und die Sonde (130) bezüglich des Zielobjekts (100) zu bewegen und gleichzeitig eine Positionsbeziehung zwischen dem optischen System und der Sonde aufrechtzuerhalten.

**12.** Signalverarbeitungsverfahren zum Bestimmen von Zielobjektinformation in einem Betrachtungsgebiet eines Zielobjekts basierend auf mehreren Empfangssignalgruppen, die jeweils mehreren Zeitpunkten entsprechen, wobei die mehreren Empfangssignalgruppen von mehreren Wandlern ansprechend auf den Empfang akustischer Wellen ausgegeben werden, die durch Bestrahlen des Zielobjekts mit Licht an einer anderen Position an einem jeweiligen der mehreren Zeitpunkten erzeugt werden, wobei das Signalverarbeitungsverfahren umfasst:

Bestimmen einer Lichtstärke im Betrachtungsgebiet für einen jeweiligen der mehreren Zeitpunkte;
Erfassen von Empfangssignalen, die der vom Betrachtungsgebiet erzeugten und durch die mehreren Wandler empfangenen akustischen Welle entsprechen für einen jeweiligen der mehreren Zeitpunkte,
Gewichten der Empfangssignale, die der vom Betrachtungsgebiet erzeugten und durch die mehreren Wandler empfangenen akustischen Welle entsprechen, mit einem Gewichtungsfaktor für einen jeweiligen der mehreren Zeitpunkte; und
Bestimmen der Zielobjektinformation im Betrachtungsgebiet basierend auf den mehreren gewichteten Empfangssignalen, die jeweils den mehreren Zeitpunkten entsprechen; und **gekennzeichnet durch**
Bestimmen des Gewichtungsfaktors basierend auf der Lichtstärke im Betrachtungsgebiet für einen jeweiligen der mehreren Zeitpunkte.

**13.** Computerprogramm, das Anweisungen umfasst, welche bei Ausführen des Programms auf einem Computer den Computer veranlassen, das Verfahren nach Anspruch 12 durchzuführen.

## Revendications

**1.** Appareil photo-acoustique, comprenant :

une unité d'exposition à un rayonnement (110, 120) configurée pour exposer un objet cible (100) à un rayonnement de lumière ;
une sonde (130) comprenant une pluralité de transducteurs (131) et configurée pour recevoir des ondes acoustiques générées à partir de l'objet cible exposé au rayonnement de lumière et pour délivrer en sortie un groupe de signaux de réception ;

une unité de mémorisation (153) configurée pour mémoriser le groupe de signaux de réception délivré en sortie par la pluralité de transducteurs ; et
une unité de traitement (150) configurée pour déterminer des informations d'objet cible dans une région d'intérêt de l'objet cible,
dans lequel l'unité d'exposition à un rayonnement est configurée pour exposer l'objet cible au rayonnement de lumière à une position différente à chacun de la pluralité d'instants,
dans lequel la sonde est configurée pour recevoir, pour chacun de la pluralité d'instants, une onde acoustique générée par une exposition de l'objet cible au rayonnement de lumière, et pour délivrer en sortie une pluralité de groupes de signaux de réception correspondant respectivement à la pluralité d'instants,
dans lequel l'unité de mémorisation est configurée pour mémoriser la pluralité de groupes de signaux de réception, et
dans lequel l'unité de traitement est en outre configurée pour :

déterminer, pour chacun de la pluralité d'instants, une fluence de lumière dans la région d'intérêt,
obtenir, pour chacun de la pluralité d'instants, des signaux de réception correspondant à l'onde acoustique générée à partir de la région d'intérêt et reçue par la pluralité de transducteurs, à partir de la pluralité de groupes de signaux de réception mémorisés dans l'unité de mémorisation,
pondérer, d'un facteur de pondération, pour chacun de la pluralité d'instants, les signaux de réception correspondant à l'onde acoustique générée à partir de la région d'intérêt et reçue par la pluralité de transducteurs, et
déterminer les informations d'objet cible dans la région d'intérêt sur la base de la pluralité de signaux de réception pondérés correspondant respectivement à la pluralité d'instants ; et **caractérisé en ce que**
l'unité de traitement est configurée pour déterminer, pour chacun de la pluralité d'instants, le facteur de pondération sur la base de la fluence de lumière dans la région d'intérêt.

2. Appareil photo-acoustique selon la revendication 1, dans lequel l'unité de traitement (150) est en outre configurée pour :

déterminer une pression sonore initiale, pour chacun de la pluralité d'instants, sur la base de la pluralité de signaux de réception pondérés,
pondérer, du facteur de pondération, la fluence de lumière dans la région d'intérêt pour chacun de la pluralité d'instants pour calculer une fluence de lumière pondérée pour chacun de la pluralité d'instants, et
déterminer les informations d'objet cible sur la base de la pression sonore initiale et de la fluence de lumière pondérée pour chacun de la pluralité d'instants.

3. Appareil photo-acoustique selon la revendication 1 ou 2, dans lequel le facteur de pondération est la fluence de lumière dans la région d'intérêt.

4. Appareil photo-acoustique selon l'une quelconque des revendications 1 à 3, dans lequel le facteur de pondération est 0 dans un cas dans lequel la fluence de lumière dans la région d'intérêt est inférieure à une valeur seuil.

5. Appareil photo-acoustique selon l'une quelconque des revendications 1 à 4,
dans lequel l'unité de traitement (150) est en outre configurée pour obtenir des informations concernant une région dans laquelle l'objet cible (100) est exposé à un rayonnement de lumière désigné par un utilisateur, et
déterminer la fluence de lumière dans la région d'intérêt, sur la base des informations concernant la région dans laquelle l'objet cible (100) est exposé à un rayonnement de lumière.

6. Appareil photo-acoustique selon la revendication 5,
dans lequel les informations concernant la région sont des informations de grandeur de la lumière émise par l'unité d'exposition à un rayonnement (110, 120), et
dans lequel l'unité de traitement (150) est configurée pour déterminer la fluence de lumière dans la région d'intérêt sur la base des informations de la grandeur de la lumière par une détermination d'une région étendue par extension d'une région ayant la grandeur de la lumière dans une direction de rayonnement et par une détermination du fait que la fluence de lumière à l'intérieur de la région étendue est 1 et que la fluence de lumière à l'extérieur de la région étendue est 0.

7. Appareil photo-acoustique selon l'une quelconque des revendications 1 à 5, dans lequel l'unité de traitement (150) est en outre configurée pour obtenir une fluence de lumière dans la région d'intérêt, au moyen d'une équation de transport ou d'une équation de diffusion sur la base de caractéristiques d'absorption optique et de caractéristiques de diffusion de lumière de l'objet cible (100).

8. Appareil photo-acoustique selon l'une quelconque des revendications 1 à 7, dans lequel la sonde (130) est formée de façon à comporter une surface sphérique, et la pluralité de transducteurs (131) sont situés sur la surface sphérique.

9. Appareil photo-acoustique selon l'une quelconque des revendications 1 à 8, dans lequel l'unité de traitement est en outre configurée pour amener une unité d'affichage (160) à afficher les informations d'objet cible.

10. Appareil photo-acoustique selon l'une quelconque des revendications 1 à 9, dans lequel l'unité d'exposition à un rayonnement comprend une source de lumière (110), un système optique (120) configuré pour guider de la lumière provenant de la source de lumière dans l'objet cible (100), et une unité de déplacement (140) configurée pour déplacer le système optique (120) par rapport à l'objet cible (100).

11. Appareil photo-acoustique selon la revendication 10, dans lequel l'unité de déplacement (140) est configurée pour déplacer le système optique (120) et la sonde (130) par rapport à l'objet cible (100) tout en conservant une relation de position entre le système optique et la sonde.

12. Procédé de traitement de signaux permettant de déterminer des informations d'objet cible dans une région d'intérêt d'un objet cible, sur la base d'une pluralité de groupes de signaux de réception correspondant respectivement à une pluralité d'instants, la pluralité de groupes de signaux de réception étant délivrée en sortie par une pluralité de transducteurs en réponse à une réception d'ondes acoustiques générées par une exposition de l'objet cible à un rayonnement de lumière à une position différente à chacun de la pluralité d'instants, le procédé de traitement de signaux comprenant les étapes consistant à :

déterminer, pour chacun de la pluralité d'instants, une fluence de lumière dans la région d'intérêt ;
obtenir, pour chacun de la pluralité d'instants, des signaux de réception correspondant à l'onde acoustique générée à partir de la région d'intérêt et reçue par la pluralité de transducteurs, à partir de la pluralité de signaux de réception ;
pondérer, d'un facteur de pondération, pour chacun de la pluralité d'instants, les signaux de réception correspondant à l'onde acoustique générée à partir de la région d'intérêt et reçue par la pluralité de transducteurs ; et
déterminer les informations d'objet cible dans la région d'intérêt sur la base de la pluralité de signaux de réception pondérés correspondant respectivement à la pluralité d'instants ; et **caractérisé par**
la détermination, pour chacun de la pluralité d'instants, du facteur de pondération sur la base de la fluence de lumière dans la région d'intérêt.

13. Programme d'ordinateur comprenant des instructions qui, lorsque le programme est exécuté par un ordinateur, amènent l'ordinateur à mettre en œuvre le procédé de la revendication 12.

[Fig. 1]

[Fig. 2]

[Fig. 3]

```
                    ( START MEASUREMENT )
                              │
        ┌─────────────────────┤
        │                     ▼
        │            ┌──────────────────────┐
        │            │    RADIATE LIGHT      │╭S100
        │            └──────────────────────┘
        │                     │
        │                     ▼
   S400 │            ┌──────────────────────┐
    ╭   │            │ RECEIVE PHOTOACOUSTIC │╭S200
 ┌──────┐            │        WAVES          │
 │ SCAN │            └──────────────────────┘
 └──────┘                     │
        ▲                     ▼
        │            ┌──────────────────────┐
        │            │ STORE RECEPTION SIGNAL│╭S300
        │            │        GROUP          │
        │            └──────────────────────┘
        │                     │
        │                     ▼
   NO   │            ◇────────────────────◇
  ◄─────┘            │ SCANNING COMPLETED?  │
                     ◇────────────────────◇
                              │ YES
                              ▼
                     ┌──────────────────────┐
                     │ ACQUIRE LIGHT FLUENCE │╭S500
                     │  IN REGION OF INTEREST│
                     └──────────────────────┘
                              │
                              ▼
                     ┌──────────────────────┐
                     │ ACQUIRE INITIAL SOUND │╭S600
                     │   PRESSURE IN REGION  │
                     │     OF INTEREST       │
                     └──────────────────────┘
                              │
                              ▼
                     ┌──────────────────────┐
                     │ ACQUIRE SUBSTANTIAL   │╭S700
                     │  LIGHT FLUENCE IN     │
                     │  REGION OF INTEREST   │
                     └──────────────────────┘
                              │
                              ▼
                     ┌──────────────────────┐
                     │ ACQUIRE ABSORPTION    │╭S800
                     │ COEFFICIENT FOR       │
                     │ REGION OF INTEREST    │
                     └──────────────────────┘
                              │
                              ▼
                     ┌──────────────────────┐
                     │ DISPLAY ABSORPTION    │╭S900
                     │ COEFFICIENT FOR       │
                     │ REGION OF INTEREST    │
                     └──────────────────────┘
                              │
                              ▼
                    ( END MEASUREMENT )
```

[Fig. 4A]

[Fig. 4B]

[Fig. 5A]

[Fig. 5B]

[Fig. 6]

[Fig. 7A]

[Fig. 7B]

[Fig. 7C]

[Fig. 7D]

[Fig. 8A]

[Fig. 8B]

[Fig. 8C]

[Fig. 9]

[Fig. 10]

[Fig. 11A]

[Fig. 11B]

**EP 3 142 543 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010022812 A
- US 2014036636 A